Europäisches Patentamt

European Patent Office

Office européen des brevets

(11) Veröffentlichungsnummer : **0 070 068**
**B1**

(12) **EUROPÄISCHE PATENTSCHRIFT**

(45) Veröffentlichungstag der Patentschrift :
21.11.85

(21) Anmeldenummer : 82200820.7

(22) Anmeldetag : 01.07.82

(51) Int. Cl.⁴ : **A 61 F   5/47**

(54) **Okklusivpessar.**

(30) Priorität : 14.07.81 CH 4615/81

(43) Veröffentlichungstag der Anmeldung :
19.01.83 Patentblatt 83/03

(45) Bekanntmachung des Hinweises auf die Patenterteilung : 21.11.85 Patentblatt 85/47

(84) Benannte Vertragsstaaten :
AT BE CH DE FR GB IT LI LU NL SE

(56) Entgegenhaltungen :
FR-A- 2 049 513
US-A- 3 405 711
US-A- 3 464 409
US-A- 3 817 248
US-A- 3 918 443

(73) Patentinhaber : Cimber, Hugo, Dr. med.
Neufeldstrasse 134
CH-3012 Bern (CH)

(72) Erfinder : Cimber, Hugo, Dr. med.
Neufeldstrasse 134
CH-3012 Bern (CH)

(74) Vertreter : Bovard, Fritz Albert et al
Bovard AG Patentanwälte VSP Optingenstrasse 16
CH-3000 Bern 25 (CH)

**Beschreibung**

Abgesehen von den üblichen Okklusivpessaren, die in der Regel als ein- oder mehrteilige, starre Körper ausgebildet sind und deshalb nur mühsam eingeführt und wieder entfernt werden können, sind Pessare bekannt, welche einen, vermittels einer entsprechenden Zuleitung aufblasbaren Füllkörper besitzen. Diese Füllkörper sind bei den vorbekannten Pessaren als in der Draufsicht dreieckiger Sack oder als Torus mit Schliessmembrane ausgebildet, wobei sie sich in aufgeblasenem Zustand mindestens gegen Teile der Gebärmutterwand abstützen und durch eine teilweise Ausfüllung der Gebärmutter eine Empfängnis einigermassen verhüten.

Pessare dieser vorbekannten Art besitzen den Nachteil, dass die gewünschte Sicherheit gegen eine Empfängnis nicht gegeben ist, und dass dazuhin die mindestens teilweise Ausfüllung der Gebärmutter dazu führt, dass das Menstruationsblut nicht abfliessen kann. Des weiteren hat der stete Druck der betreffenden aufgeblasenen Füllkörper auf die Gebärmutterschleimhaut zur Folge, dass die Gefahr der Bildung einer Drucknekrose besteht.

Um diese Nachteile zu vermeiden, wurden bereits in die Gebärmutter einzuführende Okklusivpessare vorgeschlagen, welche aus einem hohlen Stutzen und zwei von diesem seitlich abstehenden hohlen Ästen bestehen, wobei am freien Ende jedes Astes ein als kugeliger Anpreßkörper ausgebildetes Verschlußorgan angeordnet ist, wobei die Verschlußorgane dazu bestimmt und geeignet sind, ausschliesslich die Mündungen der Eileiter in die Gebärmutter zu verschliessen, und wobei der Stutzen und die beiden Äste aus steifem, formbeständigem und elastisch deformierbarem Material bestehen.

Bei diesen vorbekannten Okklusivpessaren (US-A-3 405 711) war die Verschlusswirkung der Verschlussorgane ungenügend, weil sie nämlich nicht in der Lage waren, sich gegen die Mündung des Eileiterganges wirklich dichtend anzuschmiegen. Des weiteren wurden die betreffenden Anpresskörper durch weitere Abstützkörper in ihrer Lage gehalten, was zu einer umständlichen Einführung der betreffenden Pessare führte, da auch diese Abstützkörper in den Uterus eingeführt werden mussten.

Der vorliegenden Erfindung lag nun die Aufgabe zugrunde, ein Okklusivpessar der vorstehenden Art so auszubilden, dass einerseits der dichte Abschluss der Eileitermündungen verbessert und andererseits die Plazierung der Verschlussorgane an die richtige Stelle wesentlich vereinfacht wird. Eine solche Anordnung führt zu einer entscheidenden Erleichterung des Einführens des Pessars in die Gebärmutter.

Dies wird erfindungsgemäss dadurch erreicht, dass die aus einer dünnen Folie, z. B. aus Silikon-Kautschukfolie, bestehenden Verschlussorgane zu einer Kugel aufblasbar sind, und dass das Okklusivpessar ausschliesslich von den Anpresskörpern in der Gebärmutter gehalten wird. Der hohle Stutzen kann an seinem freien Ende durch eine durchstechbare Membrane abgeschlossen sein.

In der Zeichnung ist eine beispielsweise Ausführungsform des Erfindungsgegenstandes dargestellt, und zwar zeigen die

Figuren 1 und 2 zwei mögliche Ausführungsbeispiele des erfindungsgemässen Okklusivpessars in schaubildlicher Darstellung ;

Figur 3 einen Schnitt durch das Aussenrohr mit eingezogenem Pessar ;

Figur 4 einen Schnitt durch das Innenrohr und das Aussenrohr bei ausgestossenem Pessar und

Figur 5 den Pessar, teilweise geschnitten, in seiner Wirkstellung mit eingeschobener Nadel samt Katheter und Spritze.

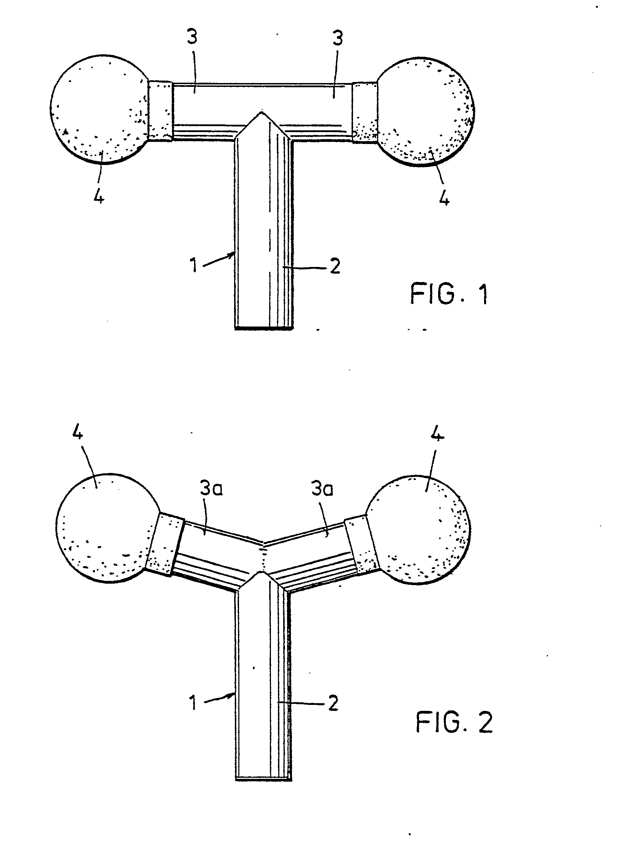

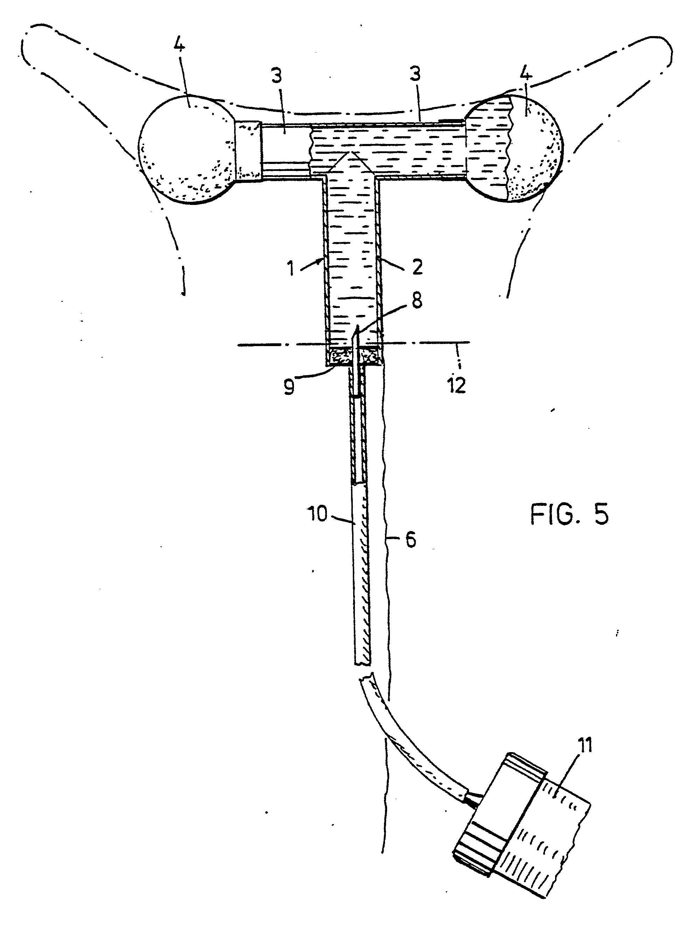

Das erfindungsgemässe Pessar gemäss einem ersten Ausführungsbeispiel nach Fig. 1 besitzt einen im wesentlichen T-förmigen Tragkörper aus einem elastischen, aber steifen und formbeständigen Rohr aus Silikonkautschuk. Der Tragkörper 1 besteht aus einem Stutzen und zwei zu verschiedenen Seiten rechtwinklig vom Stutzen abstehenden Ästen 3, so dass der Tragkörper 1 in der in den Fig. 1 und 5 dargestellten Lage im wesentlichen eine T-Form besitzt.

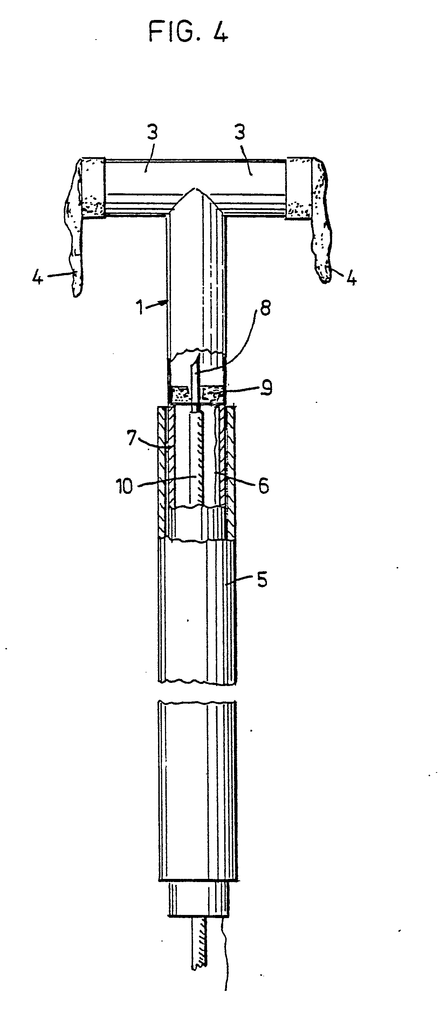

Am Ende jedes Astes 3 ist ein ballonförmiger Anpresskörper 4 aus einer dünnen Silikonkautschukfolie aufvulkanisiert, so dass, wenn durch den Stutzen 2 ein Druckmedium eingeführt wird, die beiden Anpresskörper 4 sich, wie z. B. in Fig. 1 dargestellt, zu Ballonen aufblasen lassen, während sie bei Wegfall eines Innendruckes schlaff an den Aussenenden der beiden Äste 3 herabhängen. (vgl. Fig. 4).

Die zweite Ausführungsform gemäss Fig. 2 unterscheidet sich von der ersten dadurch, dass die geometrischen Achsen der beiden Äste 3a zusammen einen Winkel einschliessen.

Es besteht auch die Möglichkeit, den Stutzen 2 zu verkupfern, um auf diese Weise eine Doppelwirkung zu erzielen, nachdem die empfängnisverhütende Wirkung des betreffenden Metalles bekannt ist.

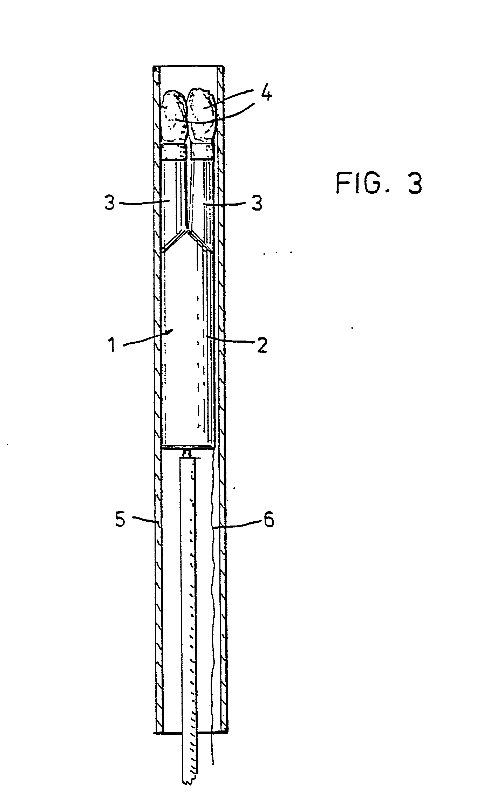

Fig. 3 zeigt ein Ausführungsbeispiel des Erfindungsgegenstandes gemäss Fig. 1 oder Fig. 2 in einer unwirksamen Lage, in welcher der Tragkörper 1 (beispielsweise mittels des Fadens 6) in ein Aussenrohr 5 eingezogen ist. Die beiden Äste 3 liegen dabei praktisch parallel zueinander sowie zum Stutzen 2 innerhalb des Aussenrohres 5.

Zur Einführung des Tragkörpers 1 wird in das Aussenrohr 5 ein Innenrohr 7 eingeschoben (vgl. Fig. 4), welches sich gegen die Unterseite des Stutzens 2 abstützt. Je nach den örtlichen Gegebenheiten wird schon vor dem Einführungsvorgang gemäss Fig. 4 eine Hohlnadel 8 in die den Stutzen 2 unten abschliessende Membrane 9 eingesteckt, welche Hohlnadel 8 über einen Katheter 10 mit einer Druckmediumquelle, beispielsweise einer Spritze 11, verbunden ist.

Sobald der Tragkörper in die in Fig. 4 dargestellte Lage vorgeschoben ist und sich innerhalb der Gebärmutter befindet, schwenken die Äste 3 bzw. 3a dank ihrer Eigenfederung in die in Fig. 1 bzw. Fig. 2 dargestellte Lage aus. Anschliessend wird vermittels der Spritze 11 ein Druckmedium, z. B. Wasser, in den Tragkörper 1 eingegeben, unter dessen Einfluss sich die Anpresskörper 4 ballonförmig aufblähen und sich auf die Mündungen der Eileiter in die Gebärmutter zum Zwecke der Bildung eines dichten Verschlusses auflegen.

Wird als Druckmedium eine Flüssigkeit verwendet, so hat es sich als vorteilhaft erwiesen, diese zu färben, um auf diese Weise irgendwelche Leckstellen unverzüglich zu erkennen.

Dadurch, dass die Anpresskörper 4 nur im Bereich der in Fig. 5 schematisch und gestrichelt dargestellten Mündung der Eileiter mit der Gebärmutterinnenwand in Berührung kommen, wird in an sich bekannter Weise einerseits der Abfluss des Menstruationsblutes offengehalten und andererseits die Gefar einer Drucknekrose vermieden.

Dazuhin gestattet aber die nach Einführung des Druckmediums feststehende gegenseitige Stellung der beiden Äste 3 bzw. 3a, den Tragkörper 1 auf solche Weise in die Gebärmutter einzuführen, dass die Auffindung der richtigen Auflagestellen wesentlich erleichtert wird, d. h. dass es auf einfache Weise gelingt, die ballonförmigen Anpresskörper 4 mit grösserer Sicherheit im Bereich der Einmündung der Eileiter zur Auflage zu bringen, als dies mit den bis anhin bekannten Pessaren der Fall war.

Im unteren Teil bzw. am freien Ende des Stutzens 2 ist, wie bereits erwähnt, der Faden 6 befestigt, dank dessen es gelingt, den Stutzen 2 um einen solchen Betrag aus dem Muttermund herauszuziehen, dass derjenige Teil des Stutzens 2, der der Befestigung der Membrane 9 dient, abgeschnitten werden kann. Die Schnittebene ist in Fig. 5 strichpunktiert angedeutet und mit « 12 » bezeichnet. Damit tritt das Druckmedium aus dem Tragkörper 1 und den Anpresskörpern 4 wieder aus, so dass diese letzten erschlaffen, und der gesamte Tragkörper, beispielsweise vermittels einer Pincette, ohne Schwierigkeit entfernt werden kann.

## Patentansprüche

1. In die Gebärmutter einzuführendes Okklusivpessar, bestehend aus einem hohlen Stutzen (2) und zwei von diesem seitlich abstehenden, hohlen Ästen (3 ; 3a), wobei am freien Ende jedes Ästes (3 ; 3a) ein als kugeliger Anpresskörper ausgebildetes Verschlussorgan (4) angeordnet ist, wobei die Verschlussorgane (4) dazu bestimmt und geeignet sind, ausschliesslich die Mündungen der Eileiter in die Gebärmutter zu verschliessen, und wobei der Stutzen (2) und die beiden Äste (3 ; 3a) aus steifem, formbeständigem und elastisch deformierbarem Material bestehen, dadurch gekennzeichnet, dass die aus einer dünnen Folie, z. B. aus Silikon- Kautschukfolie, bestehenden Verschlussorgane (4) zu einer Kugel aufblasbar sind, und dass das Okklusivpessar ausschliesslich von den Anpresskörpern in der Gebärmutter gehalten wird.

2. Okklusivpessar nach Anspruch 1, dadurch gekennzeichnet, dass der hohle Stutzen (2) an seinem freien Ende durch eine durchstechbare Membrane (9) abgeschlossen ist.

## Claims

1. Occlusive pessary to be introduced into the uterus, consisting of a hollow connection piece (2) and two hollow branches (3 ; 3a) projecting laterally therefrom, a closure part (4) formed as a spherical pressing body being disposed at the free end of each branch (3 ; 3a), the closure parts (4) being intended and suited for blocking exclusively the openings of the Fallopian tubes into the uterus, and the connection piece (2) and the two branches (3 ; 3a) being made of stiff, resiliently deformable material of stable shape, characterized in that the closure parts (4) made of a thin film, e. g. of silicone rubber film, are inflatable into a sphere, and that the occlusive pessary is held in the uterus exclusively by the pressing bodies.

2. Occlusive pessary according to claim 1, characterized in that the hollow connection piece (2) is closed at its free end by a piercable diaphragm (9).

## Revendications

1. Pessaire occlusif destiné à être introduit dans l'utérus, constitué d'un manchon creux (2) et de deux branches (3, 3a) latérales creuses, rattachées à ce manchon et à l'extrémité libre de chacune desquelles est disposé un organe de fermeture (4) ayant la forme d'un corps de pression sphérique, ces organes de fermeture (4) étant destinés et conformés de manière à ne fermer que les embouchures des trompes utérines dans l'utérus, et le manchon (2) ainsi que les branches latérales (3, 3a) étant constitués d'un matériau rigide, autoportant et déformable élastiquement, caractérisé en ce que lesdits organes de fermeture (4) sont constitués d'une feuille mince, par exemple de caoutchouc de silicone et sont gonflables à une forme sphérique, et en ce que le pessaire occlusif est maintenu dans l'utérus uniquement par les corps de pression.

2. Pessaire occlusif selon la revendication 1, caractérisé en ce que le manchon creux (2) est fermé à son extrémité libre par une membrane (9) perforable.

FIG . 1

FIG . 2

FIG. 3

# FIG. 4

FIG. 5